# EUROPÄISCHE PATENTANMELDUNG

(11) **EP 1 723 975 A2**
(43) Veröffentlichungstag der Anmeldung: **22.11.2006**
(21) Anmeldenummer: 06113844.2
(22) Anmeldetag: 12.05.2006
(51) Int. Cl.: A61L 27/46, A61L 27/58

(54) **Resorbierbarer, biokompatibler Formkörper und Verfahren zur Herstellung**

(30) Priorität: 19.05.2005 DE 102005024296
(71) Anmelder: BAM Bundesanstalt für Materialforschung und -prüfung, 12205 Berlin (DE)
(72) Erfinder: Berger, Geord, 16341, Panketal (DE); Neumann, Gert, 13127 Berlin (DE)
(74) Vertreter: Walter, Wolf-Jürgen

(57) **Zusammenfassung**

Die Erfindung betrifft einen resorbierbaren, biokompatiblen Formkörper sowie ein Herstellungsverfahren dafür. Der Formkörper ist mechanisch bearbeitbar und umfasst eine bei <40 °C feste, biokompatible, in Wasser netzwerkbildende, organische Komponente, die in Körpern von Säugern innerhalb von 3 bis 20 Tagen resorbierbar ist, und eine darin verteilte, feste anorganische Komponente mit einer Teilchengröße d₅₀ von 5-100 nm, die in Körpern von Säugern innerhalb von 7 bis 180 Tagen resorbierbar ist. Er wird erhalten durch Umsetzen eines Sols aus Ca-Verbindungen mit einem sauren Phosphorsäureester, Dotierung des Sols mit weiteren Kationen durch Zusatz von 0 bis 5 Gew.-% Alkoholate oder Carboxylate von Na, K, Zn, Mg oder Gemischen davon, Vermischen des Sols mit der gelösten organischen Komponente, Sprühtrocken des Gemisches und Pressen des erhaltenen Granulats zu einem Formkörper.

## Beschreibung

Die Erfindung betrifft einen resorbierbaren, biokompatiblen Formkörper sowie ein Herstellungsverfahren dafür.

Es ist bekannt, Formkörper aus Gelatine herzustellen und sie in chirurgischen Verfahren beispielweise bei Hüftgelenksimplantationen, als resorbierbaren Verschluss von Röhrenknochen gegenüber eintretendem Blut und Geweberesten einzusetzen. Weitere bekannte Einsatzgebiete bei denen Formkörper aus Gelatine intracorporal eingesetzt werden, sind die Zahnheilkunde und auch die Unfallchirurgie. Nachteilig in diesen Anwendungsfällen sind ein sehr geringer Elastizitätsmodul des Materials und damit schlechte Bearbeitbarkeit sowie seine außerordentlich kurze Auflösungszeit, was sich meist auf weniger als 3 Tage beläuft.

Weiterhin ist bekannt, kristalline Calciumphosphate zusammen mit Proteinen oder Proteinhydrolysaten, u.a. auch mit Gelatine als strukturierte Komposite flüssig oder in Pastenform insbesondere als Mund- oder Zahnpflegemittel einzusetzen (DE 199 30 335 oder DE 103 40 542 A1). Die anorganischen Phosphate werden dabei aus wässrigen Lösungen ausgefällt.

Ein chirurgisches Hilfsmittel in Form von Fäden oder dünnen plastifizierbaren Blättchen aus Gelatine und Glycerin mit darin enthaltenem keramischen Hydroxylapatit mit Korngrößen von 0,5-1,8 mm ist in der US 5292349 beschrieben.

Die Erfindung stellt sich die Aufgabe, die Stabilität und Bearbeitbarkeit biologisch resorbierbarer Formkörper zu verbessern, einen entsprechenden Formkörper bereitzustellen und dadurch zugleich ein ungehindertes Einsprießen von körpereigenem Gewebe in den Formkörper bei gleichzeitigem biologischen Abbau des Formkörpers zu ermöglichen, wobei die Zeit des Abbaus an den Verlauf des Aufbaus der körpereigenen Substanz angepasst werden soll.

Erfindungsgemäß bereitgestellt wird ein resorbierbarer, biokompatibler Formkörper, umfassend eine im Temperaturbereich unter 40 °C feste, biokompatible, in Wasser netzwerkbildende, organische Komponente, die in Körpern von Säugern innerhalb von 3 bis 20 Tagen resorbierbar ist, und eine darin verteilte, feste anorganische Komponente mit einer Teilchengröße d₅₀ von 5-100 nm, die in Körpern von Säugern innerhalb von 7 bis 60 Tagen resorbierbar ist, wobei der Anteil der anorganischen Komponente im Bereich von 10-90 Gew.-% liegt, der Anteil der organischen Komponente im Bereich von 90-10 Gew.-% liegt, bezogen auf das Gesamtgewicht des Formkörpers, und der Formkörper mechanisch bearbeitbar ist und eine Druckfestigkeit von 5 bis 35 N/mm² hat.

Gegenstand der Erfindung ist auch ein Formkörper mit den genannten Merkmalen,
erhältlich durch
(a) Lösen von Calciumverbindungen oder Calciumverbindungen und wenigstens einer weiteren Metallverbindung in einem oder mehreren Lösungsmittel(n), und
(b) teilweise oder vollständige Entfernung des Wassers,
(c) Umsetzung des erhaltenen Sols mit einem sauren Phosphorsäureester der Formel (I) oder (Ia) oder einem Gemisch davon 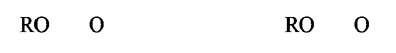 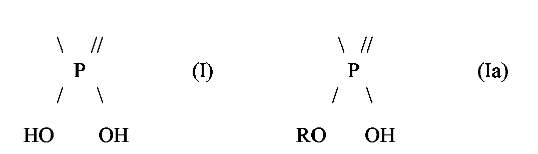 worin R die Bedeutung C₁-C₁₅-Alkyl oder Aryl hat und worin R in Formel (Ia) gleich oder verschieden sein kann und worin die Alkyl- oder Arylreste durch Hydroxy und/oder Halogen substituiert sein können, und
(d) Dotierung des erhaltenen Sols mit weiteren Kationen durch Zusatz von 0 bis 5 Gew.-% Alkoholate oder Carboxylate von Na, K, Zn, Mg oder Gemischen davon;
(e) Vermischen des flüssigen Produktes von (d) mit der organischen Komponente, die in Wasser oder organischem Lösungsmittel oder Gemisch von Wasser/organischem Lösungsmittel gelöst ist;
(f) Sprühtrocken des Gemisches von (e) mit einer für die Sprühtrocknung geeigneten Viskosität von 1-3500 mPa·s bei einer Temperatur im Bereich von 80 bis 160 °C; und
(g) Pressen des erhaltenen Granulats zu Formkörpern mit einem Druck von 1 bis 100 kN.

Dotierende Kationen werden vorzugsweise mit 0,1-5 Gew.-% eingesetzt, insbesondere 0,5-3 Gew.-%.

Der erfindungsgemäße Formkörper aus relativ schnell abbaubaren organischen Bestandteilen und etwas langsamer abbaubaren und chemisch den Körperbestandteilen nahezu identischen Calciumphosphaten bietet die Möglichkeit, einen temporären Knochenersatz mit sehr guten mechanischen Eigenschaften und definierter Resorbierbarkeit herzustellen. Diese definierte Resorbierbarkeit ist einstellbar, indem die schnelle Abbaubarkeit der organischen Komponente gegebenenfalls verzögert werden kann durch Hydrophobierung, und die etwas langsamere Abbaubarkeit der anorganischen Komponente kann durch Wahl der Teilchengröße und der entsprechenden Calciumverbindungen beschleunigt werden. Darüber hinaus ist die Resorbierbarkeit auch von bestimmten physikalischen Gegebenheiten des Einsatzortes abhängig, wie die zu resorbierende Gesamtmasse, Oberflächen, individuelle Spezifika des Patienten etc.

Überraschenderweise übertreffen die Anfangsfestigkeiten des erfindungsgemäßen Formkörpers die Anfangsfestigkeiten von üblichen abbaubaren Materialien auf Basis von Polyglykolaten bzw. die von Polylactiden. Weiterhin entstehen im Gegensatz zu den vorhergenannten bei der Resorption der erfindungsgemäßen Formkörper im Organismus keine sauren Abbauprodukte, was ebenfalls eine deutliche Verbesserung gegenüber den bekannten Produkten darstellt. In der Literatur gibt es Hinweise darauf, dass saure Abbauprodukte die Osteoclastenaktivität, d.h. also einen unerwünschten Knochenabbau begünstigen.

Aufgrund der hydrolytischen Stabilität der organischen Komponente sind keine wesentlichen Einschränkungen beim Umgang mit dem Material gegeben, so dass eine unkomplizierte Bearbeitung bei normaler Luftfeuchtigkeit möglich ist. Der Formkörper ist daher mechanisch bearbeitbar, d.h. zu sägen, zu schleifen, zu bohren usw.

Die sehr geringe Teilchengröße der anorganischen nichtkristallinen bzw. nanokristallinen Komponente ermöglicht eine Auflösung im Gewebekontakt über einen verlängerten Zeitraum auch solcher sonst schwer oder unlöslichen Calciumphosphatverbindungen und damit Ersatz durch körpereigenes Material in der vorgegebenen Form. Durch fachmännische Einstellung der prozentualen Anteile von organischer und anorganischer Komponente sowie in Abhängigkeit zu den oben genannten Bedingungen können relativ genaue Resorbierbarkeitszeiträume bei anfänglich guter Bearbeitbarkeit der Formkörper eingestellt werden.

Bevorzugte Resorbierbarkeiten liegen im Bereich von 15-100 Tagen, besonders bevorzugt 6- 60 Tagen, insbesondere 7-40 Tagen.

Unter nanokristallinen Hydroxylapatiten (HA) oder Fluorapatiten (FA), β-TCT (Tricalciumphosphat), Calcium-Alkali-Orthophosphaten wie Ca₂KNa(PO₄)₂, Ca₁₀[K/Na] (PO₄)₇, Calcium-Alkali-Metaphosphaten werden Teilchengrößen im Bereich von 7 bis 800 nm verstanden.

In der ersten Stufe zur Herstellung des erfindungsgemäßen Formkörpers werden Calciumverbindungen, z.B. anorganische Salze oder organische Calciumverbindungen gelöst. Das Lösungsmittel kann Wasser, ein organisches Lösungsmittel oder ein Gemisch davon sein. So können z.B. Calciumcarboxylate in Gemischen mit aus Essigsäure und Wasser gemeinsam mit Alkalicarboxylaten gelöst werden, so dass nach Zusatz des sauren Phosphorsäureesters nanoskalige Alkali-Erdalkaliphosphate hergestellt werden können.

Generell kann das organische Lösungsmittel ein ein- oder mehrwertiger Alkohol, Essigsäure, Acetylaceton oder ein Gemisch davon sein. Bevorzugt ist als Lösungsmittel ein C₁-C₉-Alkanol, Glycol, Glycerin oder ein Gemisch davon.

Neben diesen gelösten Calciumverbindungen können als weitere Metallverbindungen solche von Natrium, Kalium, Magnesium, Zink oder Gemische davon enthalten sein. Bevorzugt sind die Alkoholate und Carboxylate von Natrium und Kalium.

Man erhält bei diesem Verfahrensschritt Lösungen oder auch Suspensionen mit Teilchengrößen unterhalb 100 nm, die hier auch als nanoskalige Suspensionen bezeichnet werden.

Für den Fall, wo niedrige pH-Werte vermieden werden müssen, um ein stabiles Sol zu erhalten, können den Lösungen nanoskalig suspendierter anorganischer Salze organische Komplexbildner als Stabilisator zugesetzt werden. Beispielsweise gelingt es mit EDTA bei Gemischen aus Calcium- und Alkalicarboxylaten in Ethylenglycol ein Ausfallen der Bestandteile auch bei höheren Konzentrationen zu verhindern.

Spezielle bioaktive Calciumphosphatkeramiken mit bestimmten Anionen in der Art des Fluorapatits können dadurch stabilisiert werden, dass die Einführung durch Einbringung von Salzen von tertiären Aminen vorzugsweise des Triethanolamins erfolgt.

Um eine Stabilisierung der in den organischen Lösungsmitteln gelösten bzw. nanoskalig suspendierten anorganischen Salze zu erreichen, können auch nichtionogene siliziumhaltige Tenside zugesetzt werden.

Aus den nanoskaligen Suspensionen wird Wasser teilweise oder vollständig entfernt, beispielsweise durch Destillation, um ein Sol zu erhalten. Vorteilhaft kann die Destillation in Anwesenheit eines zweiten organischen Lösungsmittels erfolgen, das vorteilhaft ein einwertiger C₄-C₉-Alkohol, ein mehrwertiger Alkohol wie Glycol, Propylenglycol, Butylenglycol oder Glycerin oder ein Gemisch davon ist und das sich als Azeotrop abdestillieren lässt. Andere mit Wasser Azeotrope bildende Lösungsmittel wie Benzol, Toluol oder Xylol sind ebenfalls anwendbar.

Im Sinne der Erfindung bedeutet "Wasserentfernung" neben dem Abdestillieren oder Trockenmaßnahmen auch die Umsetzung mit z.B. Anhydriden oder die Tolerierung geringer Mengen an Kristallwasser.

Für die Erfindung ist die Entfernung von mehr oder weniger Wasser aus dem jeweiligen Gemisch der Calciumverbindungen mit den korrespondierenden Metallionen wesentlich. Dies führt in der Regel schon zu Solen, deren Bestandteile als nanoskalige Teilchen gelöst sind.

In der nächsten Verfahrensstufe wird ein Phosphorsäureester zugesetzt, der wenigstens eine freie OH-Gruppe aufweist und als "saurer" Phosphorsäureester bezeichnet wird. Damit wird ein beständiges Calciumphosphat-Sol oder Calcium-Me-phosphat-Sol erhalten. Erforderlichenfalls muss das Sol über einen bestimmten Zeitraum, wie z.B. 2-20 Stunden bei Raumtemperatur (20-25 °C) oder bei erhöhter Temperatur (26-50 °C) stehen gelassen werden bis zur Einstellung einer für den nächsten Verfahrensschritt geeigneten Viskosität. Dabei bildet sich eine gewisse Gelstruktur aus, die jedoch nicht über einer für die folgende Sprühtrocknung hinausgehenden Viskosität liegen darf. Der Fachmann kann das leicht über GFA®-Gelzeitmessung verfolgen.

Bevorzugte Viskositäten liegen im Bereich von 1-3000 mPa·s, insbesondere 10-2000 mPa·s, gemessen mit einem Rotatations-Viskosimeter, bei 25 °C.

Der saure Phosphorsäureester ist vorteilhaft ausgewählt aus der Gruppe bestehend aus einer Lösung von Phosphorpentoxid in einem C₁-C₁₆-Alkanol, einer Lösung von Phosphorpentoxid in einem gegebenenfalls durch C₁-C₁₆-Alkyl, Hydroxyalkyl oder Halogenalkyl substituierten Glycol oder einer Lösung von Phosphorpent-oxid in einem Arylalkanol, das auch substituiert sein kann.

Besonders bevorzugt als Alkanole sind Propanol und Butanol.

In den Formeln (I) und (Ia) des sauren Phosphorsäureesters ist der Alkylrest vorzugsweise ein C₁-C₄-Alkylrest, insbesondere ein C₁-C₃-Alkylrest. Der Arylrest ist vorzugsweise ein Phenyl- oder C₁-C₄-Alkylphenylrest, insbesondere ein Phenyl- oder C₁-C₃₋Alkylphenylrest, worin Alkyl den genannten bevorzugten Bedeutungen entspricht. Bevorzugte Substituenten für den Alkyl- oder Arylrest sind Hydroxy, Fluor, Chlor oder Brom.

Dem sauren Phosphorsäureester kann weiterhin Flußsäure, z.B. entwässerte HF zugegeben werden für den Fall, wo der herzustellende keramische Sinterkörper aus Fluorapatit bestehen oder diesen enthalten soll. Auch die Herstellung von Chlorapatit kann auf entsprechende Weise erfolgen.

Nach einer besonderen Ausführungsform der Erfindung kann das trockene Gelpulver einer Calcinierung bis 400 °C unterworfen werden, da meist größere Mengen an organischen Bestandteilen dem Pulver noch anhaften. Bis zu dieser Temperatur finden noch keine kristallinen Umwandlungen statt.

Die anorganische Komponente ist vorteilhaft ausgewählt aus der Gruppe, bestehend aus nanokristallinem Hydroxylapatit, nanokristallinem Fluorapatit, Tricalciumphosphat, Calciumkaliumphosphat CaKPO₄, Calciumnatriumphosphat CaNaPO₄, Gemische von Ca-Na-phosphat und Ca-K-phosphat, und Gemische mit calciumhaltigen Diphosphaten.

Besonders bevorzugt sind Calciumkaliumphosphat, Calciumnatriumphosphat, Gemische von Ca-Na-phosphat und Ca-K-phosphat und deren Gemische mit calciumhaltigen Diphosphaten. Alle genannten Phosphate und Dephosphate sind nicht-kristalline Stoffe gemäß Röntgendiffraktographie.

Das Diphosphat ist vorzugsweise Na₂CaP₂O₇, K₂CaP₂O₇, Ca₂P₂O₇ oder ein Gemisch davon.

"Resorbierbar" im Sinne der vorliegenden Erfindung bedeutet, dass im wesentlichen keine Reste des ursprünglich in das Gewebe oder in den Knochen eingebrachten erfindungsgemäßen Formkörpers mehr vorhanden sind, und dieser Formkörper zu wenigstens 95 % durch körpereigenes Material ersetzt ist.

Es ist weiterhin vorteilhaft, dass die anorganische Komponente einen Anteil reaktiver Phosphatgruppen von 10-50 Mol-% enthält, vorzugsweise 20 Mol-%. Hierdurch werden besonders starke Wasserstoffbrückenbindungen zu der organischen Komponente ausgebildet.

In dem erfindungsgemäßen Formkörper liegt der Anteil der anorganischen Komponente vorzugsweise im Bereich von 60-85 Gew.-%, insbesondere 65-85 Gew.-%. Dabei können vorteilhaft zusätzlich 0,1-1,5 Gew.-% andere anorganische Verbindungen enthalten sein, die eine weitere Anpassung der anorganischen Komponente an die natürliche Zusammensetzung des Knochens/Knorpels bzw. des Blutes gestattet, z.B. Alkalichloride, Mg-, Zn-, Si-haltige Verbindungen. Die organische Komponente hat dann einen Anteil von 40-10 Gew.-%, insbesondere 35-15 Gew.-%.

Die organische Komponente ist ein Geliermittel auf Basis von Gelatine, Cellulose oder Polysacchariden. Besonders bevorzugt ist die für organische Komponente Gelatine, Pektin oder Agar-Agar, insbesondere Gelatine. Auch Gemische von Geliermitteln können eingesetzt werden.

Bei Gelatine kann sowohl das unter sauren Bedingungen (isoelektrischer Punkt im pH-Bereich 7,5-9,3) als auch unter alkalischen Bedingungen (isoelektrischer Punkt im pH-Bereich 4,7-5,2) aus Kollagen gewonnene Produkt eingesetzt werden.

In einer bevorzugten Ausführungsform wird z.B. Gelatine vor der Vermischung mit der anorganischen Komponente acetyliert, wodurch die zur Wasserstoffbrückenbindung befähigten Aminogruppen teilweise blockiert werden und damit die Löslichkeit herabgesetzt wird. Ähnliches erfolgt bei der Acetylierung von Pektinen, wo die Löslichkeit durch Esterbildung verringert wird. Vorteilhaft wird die Acetylierung mit Essigsäureanhydrid durchgeführt. Auch eine Alkylierung kann vorher durchgeführt werden.

Gegenstand der Erfindung ist auch ein Verfahren zur Herstellung eines resorbierbaren, biokompatiblen Formkörpers durch
(a) Lösen von Calciumverbindungen oder Calciumverbindungen und wenigstens einer weiteren Metallverbindung in einem oder mehreren Lösungsmittel(n), und
(b) teilweise oder vollständige Entfernung des Wassers,
(c) Umsetzung des erhaltenen Sols mit einem sauren Phosphorsäureester der Formel (I) oder (Ia) oder einem Gemisch davon 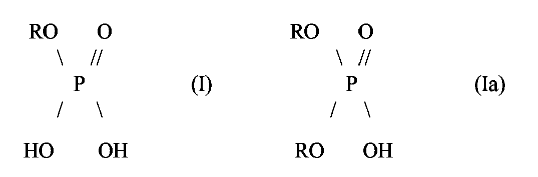 worin R die Bedeutung C₁-C₁₅-Alkyl oder Aryl hat und worin R in Formel (Ia) gleich oder verschieden sein kann und worin die Alkyl- oder Arylreste durch Hydroxy und/oder Halogen substituiert sein können,
   gegebenenfalls in Anwesenheit von HF oder HCl, und
(d) Dotierung des erhaltenen Sols mit weiteren Kationen durch Zusatz von 0 bis 5 Gew.-% Alkoholate oder Carboxylate von Na, K, Zn, Mg oder Gemischen davon;
(e) Vermischen des flüssigen Produktes von (d) mit der organischen Komponente, die in Wasser oder organischem Lösungsmittel oder Gemisch von Wasser/organischem Lösungsmittel gelöst ist;
(f) Sprühtrocken des Gemisches von (e) mit einer für die Sprühtrocknung geeigneten Viskosität im Bereich von 1-3500 mPa·s bei einer Temperatur im Bereich von 80 bis 160 °C, gegebenenfalls unter Zugabe von Arzneimitteln; und
(g) Pressen des erhaltenen Granulats zu Formkörpern mit einem Druck von 1 bis 100 kN.

Das Versprühen von noch höher viskosen Suspensionen ist durch gleichzeitige Ultraschallanwendung möglich.

In einer besonderen Ausführungsform wird das Gemisch von Stufe (e) sprühgetrocknet und während des Sprühtrocknens mit Mikrowellen mit einer Leistung von 500 W oder mehr behandelt, um eine partielle Reaktion der anorganischen mit der organischen Komponente zu erreichen.

Die Zugabe von Arzneimitteln während des Sprühtrocknens oder - bei temperaturempfindlichen Arzneimitteln - zum Granulat nach dem Sprühtrocknen kann eine vorteilhafte Maßnahme im Rahmen der Erfindung sein.

Der erfindungsgemäße Formkörper ist mechanisch fest, durch Bohren und Schleifen bearbeitbar und hat eine Druckfestigkeit von 15-22 N/mm² (Druckversuch mit ZWICK®-Gerät BDO-FB005TS)

Eine weitere bevorzugte Ausführungsform des Formkörpers besteht darin, dass der Formkörper schichtförmig aufgebaut ist mit einer äußeren Schicht, die 15-85 Gew.-% anorganische Komponente und 15-25 Gew.-% organische Komponente umfasst, und einem Innenteil, der 15-25 Gew.-% anorganische Komponente und 75-80 Gew.-% organische Komponente umfasst.

Eine weitere bevorzugte Ausführungsform des Formkörpers besteht darin, dass der Formkörper einen Konzentrationsgradienten an anorganischen und organischen Komponenten aufweist, bei dem die Konzentration der anorganischen Komponente von außen nach innen abfällt und die Konzentration der organischen Komponente von innen nach außen zunimmt.

Die Herstellung derartiger bevorzugter Ausführungsformen kann z.B. durch Aufeinanderpressen von Schichten mit unterschiedlichen Komponentenkonzentrationen erfolgen.

Die Erfindung soll nachstehend durch Beispiele und Vergleichsbeispiele näher erläutert werden. Darin sind alle Prozentangaben Gewichtsprozente.

### Beispiel 1 Hydroxylapatit

30,7 g Calciumacetat (200 mmol) werden in 30 ml Essigsäure und 30 ml Wasser gelöst. In einem weiteren Gefäß werden zur Bildung des "sauren" Esters 10,7 g reinen P₂O₅ in 80 g Butanol gelöst. Die (nanoskalige) Fällung erfolgt dann durch Zugabe des "sauren" Esters zur Calciumacetatlösung unter starkem Rühren. Teilchengröße 40-100 nm.

5g Gelatine werden in 100ml heißen Wassers von 90°C gelöst. Zu dieser Lösung werden 50ml einer wässrigen Suspension von nanoskaligem Hydroxylapatit mit einem Feststoffgehalt an 30Gew.-% gegeben und mit Ultraschall homogenisiert. Bei 150°C wird diese Lösung dann sprühgetrocknet. Um eine optimale Feuchtigkeit des gewonnenen Pulvers von 3 bis 9% einzustellen, wird in einem Vakuumtrockenschrank bei 110°C und 10mbar zwischen einer und zwei Stunden nachgetrocknet.

Zur Herstellung von Formkörpern werden in einer auf 60°C erwärmten Tablettenform (∅10mm) 2g des Pulvers eingewogen und über 5min bei 25kN verpresst.

Nach dem Abkühlen ist ein Formkörper mit guter Festigkeit entstanden, der darüber hinaus eine gute Flexibilität besitzt.

Im Druckversuch verformt sich der Zylinder bei Drücken um 20N/mm² bis zu 10% bevor die endgültige Zerstörung eintritt. Resorbierbarkeit im Tierversuch: 30-58 Tage.

### Beispiel 2 Tricalciumphosphat

18,6g Calciumacetat (100 mmol) werden in 100 ml Essigsäure und 10 ml Wasser bei 40°C gelöst. Zu dieser Lösung wird eine Lösung von 3g Gelatine in 40 ml Wasser gegeben.

Die (nanoskalige) Fällung erfolgt dann nach Zugabe von 7,7g 85%iger Phosphorsäure unter starkem Rühren durch Fällung mit 100ml 2-Propanol nach 24h bei ca. 30°C.

Beim Sprühtrocknen bei 150°C erhält man ein aggregiertes Pulver dessen Primärpartikel kleiner 1µm sind.

Die Herstellung von Formkörpern erfolgt durch Verpressen des gewonnenen Pulvers gemäß Beispiel 1 mit gleichem Ergebnis. Es wird ein bruchbeständiger und mechanisch durch Bohren, Schleifen etc. sehr gut bearbeitbarer Formkörper erhalten. Resorbierbarkeit im Tierversuch: 44-48 Tage.

### Beispiel 3 Calcium-Alkali-Orthophosphat Ca₂KNa(PO₄)₂

30g Calciumacetat, 9,8g Kaliumacetat, 13,6g Natriumacetat und 4,3g Magnesiumacetat werden in 100ml Wasser gelöst. Zur nanoskaligen Fällung werden 0,3g Hexadecyltrimethylammoniumchlorid als Tensid zugegeben und nach dem Auflösen mit 108,4g eines aus 2-Propanol und Phosphorpentoxid hergestellten sauren Esters bei 25°C gefällt.

Die Herstellung eines zum Verpressen geeigneten Pulvers erfolgt durch Sprühtrocknen nach Zugabe des in Essigsäure gelösten Binders, wobei jedoch acetylierte Gelatine eingesetzt und mit der anorganischen Komponente mit einer Teilchengröße von 50-95 nm sprühgetrocknet. Der nach dem Pressen erhaltene Formkörper wird mechanisch oberflächenbehandelt und im Tierversuch eingesetzt.
Resorbierbarkeit 28-35 Tage.

### Beispiel 4 Calciummethaphosphat, Ca(PO₃)₂

18,6g Calciumacetat (100 mmol) werden in 100 ml Essigsäure und 10 ml Wasser bei 40°C gelöst. Es erfolgt dann die(nanoskalige) Herstellung des gewünschten Pulvers nach Zugabe von 23,1g 85%iger Phosphorsäure unter starkem Rühren durch Fällung mit 100ml 2-Propanol nach 24h bei ca. 30°C. Beim Sprühtrocknen bei 150°C erhält man ein aggregiertes Pulver dessen Primärpartikel kleiner 1µm sind, wenn dem zur Fällung verwendeten Isopropanol 2% Stearinsäure zugefügt wurde. Für ein besonders schnell resorbierbares Kompositpulver werden 4g Hydroxyethylcellulose (HC) in 100ml Wasser gelöst und der Calciumphosphatsuspension zugegeben. Das sprühgetrocknete Pulver lässt sich in der beschriebenen Weise zum Formkörper verpressen.

Die durch Verpressen des Kompositpulvers hergestellten Formkörper werden im Tierversuch innerhalb von 10 bis 21 Tagen resorbiert.

Das gewünschte nanoskalige Metaphosphat erhält man aus der durch Calcinieren des ohne HC hergestellten Pulvers bei 400°C.

### Beispiel 5 Fluorapatit

30,7 g Calciumacetat (200 mmol) werden in 30 ml Essigsäure und 30 ml Wasser gelöst. In einem weiteren Gefäß werden zur Bildung des "sauren" Esters 10,7 g reinen P₂O₅ in 80 g Butanol gelöst. Weiterhin werden 2,0 g (40 mmol) Flußsäure mit 7,0 g Essigsäureanhydrid entwässert und dem "sauren" Ester zugegeben. Die (nanoskalige) Fällung erfolgt dann durch Zugabe des "sauren" Esters zur Calciumacetatlösung unter starkem Rühren. Teilchengröße 60-100 nm.

5g Gelatine werden in 100ml heißen Wassers von 90°C gelöst. Zu dieser Lösung werden 50ml einer wässrigen Suspension von nanoskaligem Fluorapatit mit einem Feststoffgehalt an 30Gew.-% gegeben und mit Ultraschall homogenisiert. Bei 150°C wird diese Lösung dann sprühgetrocknet. Um eine optimale Feuchtigkeit des gewonnenen Pulvers von 3 bis 9% einzustellen, wird in einem Vakuumtrockenschrank bei 110°C und 10mbar zwischen einer und zwei Stunden nachgetrocknet.

Zur Herstellung von Formkörpern werden in einer auf 60°C erwärmten Tablettenform (∅10mm) 2g des Pulvers eingewogen und über 5min bei 25kN verpresst.

Nach dem Abkühlen ist ein Formkörper mit guter Festigkeit entstanden, der darüber hinaus eine gute Flexibilität besitzt. Im Druckversuch verformt sich der Zylinder bei Drücken um 20N/mm² bis zu 10% bevor die endgültige Zerstörung eintritt. Resorbierbarkeit im Tierversuch: 48-72 Tage.

## Patentansprüche

1. Resorbierbarer, biokompatibler Formkörper, umfassend eine im Temperaturbereich unter 40 °C feste, biokompatible, in Wasser netzwerkbildende, organische Komponente, die in Körpern von Säugern innerhalb von 3 bis 20 Tagen resorbierbar ist, und eine darin verteilte, feste anorganische Komponente mit einer Teilchengröße d₅₀ von 5-100 nm, die in Körpern von Säugern innerhalb von 7 bis 180 Tagen resorbierbar ist, wobei der Anteil der anorganischen Komponente wenigstens 15 Gew.-% beträgt, der Anteil der organischen Komponente höchstens 85 Gew.-% beträgt, bezogen auf das Gesamtgewicht des Formkörpers, und der Formkörper mechanisch bearbeitbar ist und eine Druckfestigkeit im Bereich von 5 bis 35 N/mm² hat.

2. Formkörper nach Anspruch 1, **dadurch gekennzeichnet, dass** der Anteil der anorganischen Komponente im Bereich von 15-90 Gew.-% liegt und der Anteil der organischen Komponente im Bereich von 10-85 Gew.-% liegt.

3. Formkörper nach Anspruch 1, **dadurch gekennzeichnet, dass** die weitere Metallverbindung Natrium, Kalium, Magnesium oder ein Gemisch davon umfasst.

4. Formkörper nach Anspruch 1, **dadurch gekennzeichnet, dass** die Calciumverbindung ein alkalihaltiges Calciumphosphat ist.

5. Formkörper nach Anspruch 1, **dadurch gekennzeichnet, dass** der saure Phosphorsäureester ausgewählt ist aus der Gruppe bestehend aus einer Lösung von Phosphorpentoxid in einem C₁-C₁₆₋Alkanol, einer Lösung von Phosphorpentoxid in einem C₁-C₁₆₋alkoxylierten Glycol oder einer Lösung von Phosphorpentoxid in einem Arylalkanol.

6. Formkörper nach Anspruch 1, **dadurch gekennzeichnet, dass** die anorganische Komponente ausgewählt ist aus der Gruppe bestehend aus nanokristallinem Hydroxylapatit, nanokristallinem Fluorapatit, Tricalciumphosphat, Calciumkaliumphosphat, Calciumnatriumphosphat, Gemische von Ca-Na-phosphat und Ca-K-phosphat, Calciummetaphosphat, Calcium-Alkali-Metaphosphate und Gemische mit calciumhaltigen Diphosphaten, vorzugsweise Calciumkaliumphosphat, Calciumnatriumphosphat, Gemische von Ca-Na-phosphat und Ca-K-phosphat und deren Gemische mit calciumhaltigen Diphosphaten.

7. Formkörper nach Anspruch 6, **dadurch gekennzeichnet, dass** das Diphosphat Na₂CaP₂0₇, K₂CaP₂O₇, Ca₂P₂O₇ oder ein Gemisch davon ist.

8. Formkörper nach Anspruch 1, **dadurch gekennzeichnet, dass** der Anteil der anorganischen Komponente im Bereich von 60-90Gew.-%, insbesondere 65-85Gew.-% liegt.

9. Formkörper nach Anspruch 1, **dadurch gekennzeichnet, dass** die organische Komponente Geliermittel auf Basis von Gelatine, Cellulose oder Polysacchariden umfasst.

10. Formkörper nach Anspruch 9, **dadurch gekennzeichnet, dass** die organische Komponente Gelatine ist.

11. Verfahren zur Herstellung eines resorbierbaren, biokompatiblen Formkörpers nach Anspruch 1, **gekennzeichnet durch**
(a) Lösen von Calciumverbindungen oder Calciumverbindungen und wenigstens einer weiteren Metallverbindung in einem oder mehreren Lösungsmittel(n); und
(b) teilweise oder vollständige Entfernung des Wassers; und
(c) Umsetzung des erhaltenen Sols mit einem sauren Phosphorsäureester der Formel (I) oder (Ia) oder einem Gemisch davon 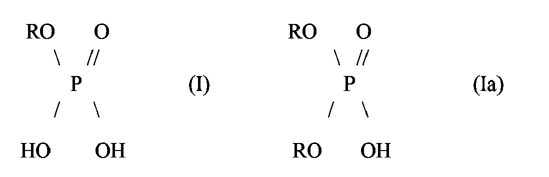 worin R die Bedeutung C₁-C₁₅-Alkyl oder Aryl hat und worin R in Formel (Ia) gleich oder verschieden sein kann und worin die Alkyl- oder Arylreste **durch** Hydroxy und/oder Halogen substituiert sein können, gegebenenfalls in Anwesenheit von HF oder HCl; und
(d) Dotierung des erhaltenen Sols mit weiteren Metallkationen **durch** Zusatz von 0 bis 5 Gew.-% Alkoholate oder Carboxylate von Na, K, Zn, Mg oder Gemischen davon;
(e) Vermischen des flüssigen Produktes von (d) mit der organischen Komponente, die in Wasser oder organischem Lösungsmittel oder Gemisch von Wasser/organischem Lösungsmittel gelöst ist; und
(f) Sprühtrocken des Gemisches von (e) mit einer für die Sprühtrocknung geeigneten Viskosität im Bereich von 1-3500 mPa·s bei einer Temperatur im Bereich von 80 bis 160 °C, gegebenenfalls unter Zugabe von Arzneimitteln; und
(g) Pressen des erhaltenen Granulats zu Formkörpern mit einem Druck von 1 bis 100 kN.

12. Verfahren nach Anspruch 11, **dadurch gekennzeichnet, dass** man das Gemisch von Stufe (e) sprühtrocknet und während des Sprühtrocknens mit Mikrowellen behandelt.

13. Verfahren nach Anspruch 11, **dadurch gekennzeichnet, dass** man eine acetylierte oder methylierte Gelatine als organische Komponente einsetzt.

14. Verfahren nach Anspruch 11, **dadurch gekennzeichnet, dass** die Entfernung von Wasser gemäß Stufe (b) mittels Destillation gegebenenfalls in Anwesenheit eines zweiten organischen Lösungsmittels erfolgt.

15. Verfahren nach Anspruch 11, **dadurch gekennzeichnet, dass** man als anorganische Komponente eine solche mit einem Anteil reaktiver Phosphatgruppen von 10-50 Mol-% einsetzt, vorzugsweise 20 Mol-%.
